# EUROPÄISCHE PATENTSCHRIFT

(11) **EP 4 373 810 B1**
(45) Veröffentlichungstag und Bekanntmachung des Hinweises auf die Patenterteilung: **08.04.2026**
(21) Anmeldenummer: 22740769.9
(22) Anmeldetag: 14.06.2022
(51) Int. Cl.: C07D 219/08, B60C 1/00, C08K 5/3437, C08L 21/00, C09B 15/00

(54) **VERBINDUNG, KAUTSCHUKMISCHUNG ENTHALTEND DIE VERBINDUNG, FAHRZEUGREIFEN, DER DIE KAUTSCHUKMISCHUNG IN WENIGSTENS EINEM BAUTEIL AUFWEIST, VERFAHREN ZU DEREN HERSTELLUNG SOWIE VERWENDUNG DER VERBINDUNG ALS ALTERUNGSSCHUTZMITTEL UND/ODER OZONSCHUTZMITTEL UND/ODER FARBSTOFF**
COMPOUND, RUBBER BLEND CONTAINING THE COMPOUND, VEHICLE TIRE COMPRISING THE RUBBER BLEND IN AT LEAST ONE COMPONENT, PROCESS FOR PRODUCING THE COMPOUND, AND USE OF THE COMPOUND AS AN AGEING PROTECTANT AND/OR ANTIOZONANT AND/OR DYE
COMPOSÉ, MÉLANGE DE CAOUTCHOUC CONTENANT LE COMPOSÉ, PNEU DE VÉHICULE COMPRENANT LE MÉLANGE DE CAOUTCHOUC DANS AU MOINS UN COMPOSANT, PROCÉDÉ DE PRODUCTION DU COMPOSÉ, ET UTILISATION DU COMPOSÉ COMME AGENT DE PROTECTION CONTRE LE VIEILLISSEMENT ET/OU AGENT ANTIOZONANT ET/OU COLORANT

(30) Priorität: 23.07.2021 DE 102021207929
(43) Veröffentlichungstag der Anmeldung: 29.05.2024
(73) Patentinhaber: Continental Reifen Deutschland GmbH, 30175 Hannover (DE)
(72) Erfinder: JACOB, Andreas, 30165 Hannover (DE); DAUER, David-Raphael, 30165 Hannover (DE); STROHMEIER, Julian, 30165 Hannover (DE); WITTENBERG, Elisabeth, 30165 Hannover (DE)
(74) Vertreter: Continental Corporation
(86) Internationale Anmeldenummer: PCT/DE2022/200128
(87) Internationale Veröffentlichungsnummer: WO 2023/001338

(56) Entgegenhaltungen:
- CN-A- 113 072 741
- DE-A1- 102019 212 916

## Beschreibung

Die Erfindung betrifft eine Verbindung, eine Kautschukmischung enthaltend die Verbindung, einen Fahrzeugreifen, der die Kautschukmischung in wenigstens einem Bauteil aufweist, Verfahren zu deren Herstellung sowie die Verwendung der Verbindung als Alterungsschutzmittel und/oder Ozonschutzmittel und/oder Farbstoff.

Es ist bekannt, dass in Fahrzeugreifen und technischen Gummiartikeln polymere Materialien, wie insbesondere Kautschuke, eingesetzt werden.

Naturkautschuk, synthetische Polymere (wie IR, BR, SSBR, ESBR, etc.) aber auch natürliche sowie synthetische Öle, Fette und Schmiermittel unterliegen bei längerer Lagerung und vor allem in der Zielanwendung, die oft bei höheren Temperaturen abläuft, Oxidationsreaktionen, die sich nachteilig auf die ursprünglichen gewünschten Eigenschaften auswirken. Je nach Art des Polymers werden die Polymerketten, bis hin zu einer Verflüssigung des Materials, verkürzt oder es kommt zur nachträglichen Härtung des Werkstoffes.

Alterungsschutzmittel tragen daher maßgeblich zur Langlebigkeit von Fahrzeugreifen und anderen technischen Gummiartikeln bei. Bekannte Alterungsschutzmittel sind aromatische Amine, wie beispielsweise 6-PPD (N-(1,3-Dimethylbutyl)-N'-phenyl-p-phenylendiamin), IPPD (N-Isopropyl-N'-phenyl-p-phenylendiamin) oder SPPD (N-(1-phenylethyl)-N'-phenyl-p-phenylendiamin).

Diese Moleküle können mit Sauerstoff oder Ozon oder gebildeten Radikalen, wie Alkyl-, Alkoxy- und Alkylperoxyradikalen, reagieren und diese somit abfangen und somit die Kautschuke etc. vor weiteren Oxidationsreaktionen schützen.

Nachteilig an dieser Substanzklasse ist allerdings der Verdacht, dass diese krebserregend sein könnten.

CN 113 072 741 A und DE 10 2019 212916 A1 offenbaren jeweils para-Phenylendiamine als Alterungsschutzmittel in Kautschukmischungen.

Alterungsschutzmittel, die insbesondere mit Ozon reagieren und dieses abfangen, werden auch als "Ozonschutzmittel" oder "Antiozonant" bezeichnet.

Der Erfindung liegt die Aufgabe zugrunde, eine neuartige Verbindung bereitzustellen, die insbesondere als Alterungsschutzmittel in Fahrzeugreifen oder anderen technischen Gummiartikeln verwendet werden kann und zwar mit einem geringeren Gefahrenpotential bei hinreichender Löslichkeit in der jeweiligen Matrix, beispielsweise und insbesondere im Polymer. Hierdurch soll unter Verringerung der Gesundheitsschädlichkeit ein weiterhin optimaler Schutz vor Sauerstoff und Ozon gewährleistet werden sowie die Tendenz zum Ausblühen (engl. "Blooming") verhindert werden.

Gelöst wird die Aufgabe durch die erfindungsgemäße Verbindung gemäß Anspruch 1, die erfindungsgemäße Kautschukmischung enthaltend die Verbindung sowie den erfindungsgemäßen Fahrzeugreifen, der die erfindungsgemäße Kautschukmischung in wenigstens einem Bauteil aufweist. Ferner wird die Aufgabe durch die Verwendung der Verbindung als Alterungsschutzmittel und/oder Ozonschutzmittel gelöst.

Die Verbindung gemäß Anspruch 1 kann ferner als Farbstoff verwendet werden. Ferner wird die Aufgabe durch erfindungsgemäße Verfahren zur Herstellung der erfindungsgemäßen Verbindung gelöst.

Die Verbindung gemäß Anspruch 1 weist die allgemeine Formel I) auf: 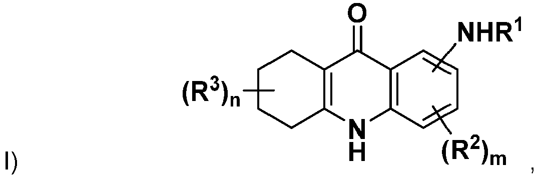
wobei R¹ ausgewählt ist aus der Gruppe bestehend aus Benzyl-, 1-Phenylalkyl-Resten mit insgesamt 7 bis 18 Kohlenstoffatomen und linearen, verzweigten und cyclischen aliphatischen C₃- bis C₁₂-Resten; und
wobei R³ ausgewählt ist aus der Gruppe bestehend aus linearen, verzweigten und cyclischen aliphatischen C₁ bis C₁₂-Resten, sowie Aryl-Resten, Ether-Resten und Thioether-Resten, und wobei n den Wert 0 oder 1 oder 2 oder 3 oder 4 annimmt,
wobei die Reste R³ im Fall von n gleich 2 oder 3 oder 4 unabhängig voneinander gleich oder verschieden sind, und
wobei R² ausgewählt ist aus der Gruppe bestehend aus linearen, verzweigten und cyclischen aliphatischen C₁ bis C₁₂-Resten, sowie Aryl-Resten, Ether-Resten und Thioether-Resten; und
wobei m den Wert 0 oder 1 oder 2 oder 3 annimmt, wobei die Reste R² im Fall von m gleich 2 oder 3 unabhängig voneinander gleich oder verschieden sind.

Dem Fachmann ist klar, dass im Fall von n gleich 0 (null) oder 1 oder 2 oder 3 anstelle des R³ jeweils ein weiteres Wasserstoffatom an das entsprechende Kohlenstoffatom des gesättigten Ringes gebunden ist. Ebenso sind im Fall von m gleich 0 oder 1 oder 2 alle übrigen freien Positionen am Benzolring des Gerüsts Wasserstoffatome.

Dem Fachmann ist ebenfalls klar, dass die Darstellung der Verknüpfungen von (R²)ₘ und (R³)ₙ sowie R¹HN in den jeweiligen (Benzol-)Ring des Gerüsts bedeutet, dass diese Gruppen jeweils an jeder Stelle am jeweiligen (Benzol-)Ring angeordnet sein können, außer im Fall des Benzolringes nicht jeweils zwei oder mehrere gleichzeitig an derselben Position, was im Fall des Benzolringes schon aufgrund der Vierbindigkeit des Kohlenstoffatoms ausgeschlossen wäre.

Bezeichnungen derart wie "C₃- bis C₁₂-Reste" bedeutet im Rahmen der vorliegenden Erfindung, dass Reste mit 3 bis 12 Kohlenstoffatomen gemeint sind. Unabhängig davon wird "C₁" als Bezeichnung der Position des höchstoxidierten Kohlenstoffatoms bzw. der höchsten Priorität nach der Cahn-Ingold-Prelog-Konvention (CIP) verwendet. Dem Fachmann ist im jeweiligen Kontext klar, was gemeint ist.

Die erfindungsgemäße Verbindung ist ein Tetrahydroacridin-Derivat und es ist über einen Analogieschluss zu erwarten, dass es gegenüber bekannten Alterungsschutzmitteln auf Basis von Anilin (mögliches Spaltprodukt von 6-PPD) ein geringeres Gefahrenpotential aufweist, da z. B. Carbazol als carcinogen eingestuft ist, während Tetrahydrocarbazol als nicht carcinogen eingestuft ist.

Gerade bei einer technischen Anwendung wie in Fahrzeugreifen oder anderen Gummiprodukten ist ein geringeres Gefahrenpotential ein entscheidender Vorteil, da durch Abrieb bzw. andere Abbauprozesse die Gummiinhaltsstoffe freigesetzt werden können.

Die erfindungsgemäße Verbindung weist zudem gegenüber 6-PPD eine verbesserte Schutzwirkung, insbesondere von Polymeren, gegenüber Oxidation und damit Alterung auf.

Von der Erfindung sind sämtliche vorteilhafte Ausgestaltungen, die sich unter anderem in den Patentansprüchen widerspiegeln, umfasst. Insbesondere sind von der Erfindung auch Ausgestaltungen umfasst, die sich durch Kombination unterschiedlicher Merkmale unterschiedlicher Abstufungen bei der Bevorzugung dieser Merkmale ergeben, sodass auch eine Kombination eines ersten als "bevorzugt" bezeichneten Merkmals oder im Rahmen einer vorteilhaften Ausführungsform beschriebenen Merkmals mit einem weiteren als z. B. "besonders bevorzugt" bezeichneten Merkmal von der Erfindung umfasst ist.

Bevorzugt ist n gleich 0 (null).

Bevorzugt ist m gleich 0 (null).

Es ist bevorzugt, dass R¹ über ein tertiäres Kohlenstoffatom an das Stickstoffatom (N) gebunden ist. Damit ist das C₁-Atom bevorzugt ein tertiäres Kohlenstoffatom. Unter dem Begriff "tertiäres Kohlenstoffatom" ist im Rahmen der vorliegenden Erfindung ein Kohlenstoffatom zu verstehen, welches an nur ein Wasserstoffatom gebunden ist.

Besonders bevorzugt ist R¹ ein verzweigter Alkyl-Rest mit 3 bis 12 Kohlenstoffatomen, wiederum bevorzugt 4 bis 8 Kohlenstoffatomen, oder ein 1-Phenylalkyl-Rest mit insgesamt 7 bis 10 Kohlenstoffatomen. Hierbei ist bevorzugt zumindest eine Verzweigung am C₁-Atom vorhanden, also an dem an das Stickstoffatom (N) gebundene Kohlenstoffatom, womit das C₁-Atom ein tertiäres Kohlenstoffatom ist.

Ganz besonders bevorzugt ist R¹ ausgewählt aus 1,3-Dimethylbutyl-, 1-Phenylethyl- und Cyclohexyl-Resten, wiederum ganz besonders bevorzugt ist R¹ ein 1,3-Dimethylbutyl-Rest.

In einer bevorzugten Ausführungsform weist die Verbindung die Struktur gemäß Formel II) auf:

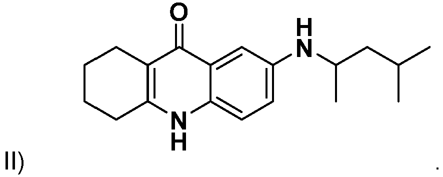

Mit der Verbindung gemäß Formel II) kann, insbesondere in Polymeren, sogar eine weitere Verbesserung des Schutzes vor Oxidation und somit Alterung erzielt werden. Gleichzeitig ist die Verbindung gemäß Formel II) deutlich weniger gesundheitsschädlich als z. B. 6-PPD oder andere Vertreter dieser Substanzklasse, wie einleitend aufgeführt.

Im Vergleich zu 6-PPD ist die Verbindung gemäß Formel II) somit ein besseres und gleichzeitig gesundheits- und umweltfreundlicheres Alterungsschutzmittel.

Die erfindungsgemäße Verbindung gemäß Formel I) bzw. Formel II) bzw. sämtlicher obiger Ausführungen ist besonders als Alterungsschutzmittel und/oder Ozonschutzmittel in Fahrzeugreifen und/oder technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen geeignet.

Ein weiterer Gegenstand der vorliegenden Erfindung ist daher die Verwendung der erfindungsgemäßen Verbindung als Alterungsschutzmittel und/oder Ozonschutzmittel in Fahrzeugreifen und/oder technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen.

Zur Verwendung der Verbindung gemäß Formel I) bzw. Formel II) bzw. sämtlicher obiger Ausführungen in den genannten Artikeln oder Stoffen wird diese in einer Zusammensetzung verwendet und in dieser eingemischt verwendet.

Bei Fahrzeugreifen oder anderen technischen Gummiartikeln ist dies insbesondere eine Kautschukmischung.

Ein weiterer Gegenstand der Erfindung ist die Verwendung der erfindungsgemäßen Verbindung gemäß Formel I) bzw. Formel II) bzw. sämtlicher obiger Ausführungen als Farbstoff in Fasern und/oder Polymeren und/oder Papier und/oder in (Streich-)Farben und Lacken.

Ein weiterer Aspekt der vorliegenden Erfindung ist ein Verfahren zur Herstellung der Verbindung gemäß Formel I), welches die folgenden Verfahrensschritte umfasst:
a1) Bereitstellung der Verbindung gemäß Formel A1) 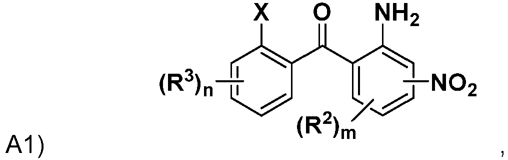 wobei für die Reste R², R³ sowie die Indizes m und n die obigen Ausführungen gelten und X ein Halogen, insbesondere Fluor (F), Chlor (Cl) oder Brom (Br), ), besonders bevorzugt Chlor oder Fluor, ganz besonders bevorzugt Fluor, ist;
b1) Umsetzung der Verbindung gemäß Formel A1) mit einer Base, insbesondere Kaliumcarbonat (K₂CO₃), wobei die Verbindung gemäß Formel B1) erhalten wird: 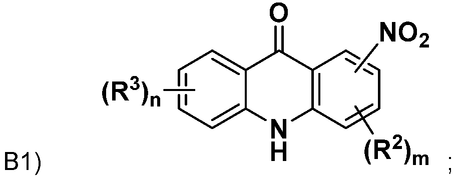
c1) Umsetzung der Verbindung gemäß Formel B1) mit Wasserstoff oder einem Hydrierungsreagenz, insbesondere einem Hydrid, und einem Keton oder Aldehyd (R¹=O), zu der Verbindung gemäß Formel I):

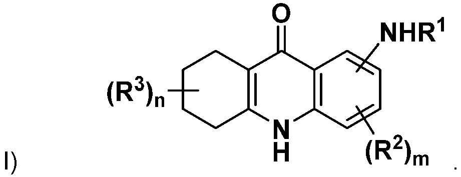

Die Base in Schritt b1) ist bevorzugt eine starke Base, wie Kaliumcarbonat (K₂CO₃) oder Kaliumphosphat (K₃PO₄). Besonders bevorzugt wird Kaliumcarbonat (K₂CO₃) verwendet.

Die Umsetzung gemäß Schritt b1) erfolgt bevorzugt in einem polaren Lösungsmittel, wie insbesondere Dimethylformaldehyd (DMF) oder Dimethylsulfoxid (DMSO). Besonders bevorzugt wird Dimethylformaldehyd (DMF) verwendet.

Weitere bevorzugte Verfahrensmerkmale werden zusammen mit bevorzugten Verfahrensmerkmalen des weiteren Verfahrens zur Herstellung der Verbindung gemäß Formel I) genannt.

Ein weiterer Gegenstand der vorliegenden Erfindung ist ein weiteres Verfahren zur Herstellung der Verbindung gemäß Formel I), welches wenigstens die folgenden Verfahrensschritte umfasst:
a2) Bereitstellung einer Verbindung gemäß Formel A2): 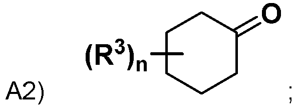 und
b2) Bereitstellung einer Verbindung gemäße Formel B2): 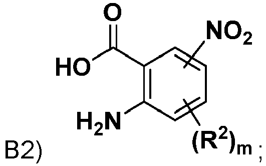 und
c2) Umsetzung der Verbindung gemäß Formel A2) mit der Verbindung gemäß Formel B2) in Gegenwart eines Halogenidüberträgers, wie PCl₃, POCl₃, PBr₃ oder SOCl₂, zu der Verbindung gemäß Formel C2): 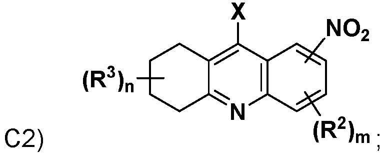 und
d2) Umsetzung der Verbindung gemäß Formel C2) in Gegenwart einer Säure, wie insbesondere Essigsäure, zu der Verbindung gemäß Formel B1): 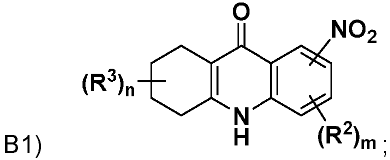 und
e2) Umsetzung der Verbindung gemäß Formel B1) mit Wasserstoff oder einem Hydrierungsreagenz, insbesondere einem Hydrid, und einem Keton oder Aldehyd (R¹=O), zu der Verbindung gemäß Formel I): 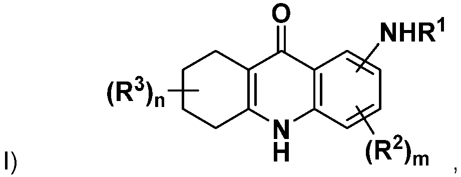 wobei für die Reste R¹, R², R³ sowie die Indizes m und n die obigen Ausführungen gelten und X ein Halogen, insbesondere Fluor (F), Chlor (Cl) oder Brom (Br), besonders bevorzugt Chlor oder Fluor, ganz besonders bevorzugt Chlor, ist.

Der Halogenidüberträger in Schritt c2) ist insbesondere ein saurer Halogenidüberträger. Der Halogenidüberträger in Schritt c2) ist bevorzugt ausgewählt aus der Gruppe bestehend aus PCl₃, POCl₃, PBr₃ und SOCl₂. Besonders bevorzugt wird PCl₃ oder POCl₃ verwendet.

Bevorzugt wird die Verbindung gemäß Formel C2) nach ihrer Isolierung, dabei wiederum bevorzugt ohne weitere Aufreinigung, in ein Druckgefäß überführt. Die Umsetzung gemäß Schritt d2) zu der Verbindung gemäß Formel D2) erfolgt bevorzugt in einem Druckgefäß, also in einem für vergleichsweise hohe Drücke geeigneten Behälter, wie insbesondere einem Autoklaven oder einem anderem Druckreaktor.

Bevorzugt erfolgt die Umsetzung gemäß Schritt d2) bei einer Temperatur von 200 bis 240 °C, insbesondere und beispielsweise 220 °C.

Die Säure in Schritt d2) ist insbesondere eine in Wasser lösliche Säure, wie insbesondere Essigsäure, und wird insbesondere in verdünnter wässriger Lösung eingesetzt, wie insbesondere mit Wasser verdünnte Essigsäure. Die Säure ist insbesondere auch eine nicht nucleophile Säure.

Unter "Hydrierungsreagenz" wird eine Verbindung verstanden, die eine Hydrierung ermöglicht. Hierzu zählen wie dem Fachmann bekannt Hydride, insbesondere Metallhydride.

Ein geeignetes Hydrid ist z. B. Natriumborhydrid.

Wasserstoff wird im Rahmen der vorliegenden Erfindung nicht zusätzlich unter "Hydrierungsreagenz" gelistet, da es explizit als Alternative genannt ist. Natürlich sind dennoch sämtliche Reagenzien unter "Hydrierungsreagenz" umfasst, die in situ Wasserstoff bilden, welches die Hydrierung bewirkt.

Bevorzugt erfolgt die Umsetzung in Schritt c1) bzw. e2) mit Wasserstoff (H₂) und dem Keton oder Aldehyd (R¹=O), bevorzugt Keton, unter Verwendung eines Hydrierungskatalysators.

Bevorzugt erfolgt die Umsetzung gemäß Schritt c1) bei einer Temperatur von 120 bis 150 °C, insbesondere zum Beispiel 140 °C.

Bevorzugt wird in Schritt c1) Wasserstoff mit einem Druck von 35 bis 45 bar, insbesondere zum Beispiel 40 bar, aufgedrückt und bevorzugt anschließend für 1 bis 20 Stunden, bevorzugt 8 bis 13 Stunden, insbesondere zum Beispiel 10 Stunden, gerührt.

Bevorzugt erfolgt die Umsetzung gemäß Schritt e2) bei einer Temperatur von 50 bis 70 °C, insbesondere zum Beispiel 60 °C.

Bevorzugt wird in Schritt e2) Wasserstoff mit einem Druck von 15 bis 25 bar, insbesondere zum Beispiel 20 bar, aufgedrückt und bevorzugt anschließend für 1 bis 20 Stunden, bevorzugt 8 bis 13 Stunden, insbesondere zum Beispiel 10 Stunden, gerührt.

Bei dem Keton in Schritt c1) bzw. e2) handelt es sich um das Keton-Derivat des späteren Restes R¹; Bei einem Aldehyd entsprechend um das Aldehyd-Derivat. Der Einfachheit halber wird verkürzt die Formel R¹=O für das Aldehyd oder Keton verwendet, da der Rest R¹ der Teil ist, der nach der Reaktion mit dem Aldehyd oder Keton am Stickstoffatom verbleibt.

Bevorzugt wird hierbei das Keton Methylisobutylketon verwendet.

Bevorzugt erfolgt die Umsetzung mit Wasserstoff in Schritt c1) bzw. e2) in einem für die vergleichsweise hohen Drücke geeigneten Behälter, wie insbesondere einem Autoklaven oder einem anderem Druckreaktor.

Bevorzugt wird in den Verfahrensschritten, in denen eine Umsetzung mit Wasserstoff erfolgt, ein geeigneter Katalysator, im Rahmen der vorliegenden Erfindung als "Hydrierungskatalysator" bezeichnet, verwendet.

Bevorzugt ist der Hydrierungskatalysator ein Edelmetallkatalysator, wie insbesondere Palladium (Pd) oder Platin (Pt). Bevorzugt wird das Edelmetall auf Kohle (C) eingesetzt, wie Palladium auf Kohle (Pd/C).

Ferner können auch andere bekannte Katalysatoren, wie Raney-Nickel oder Kupferchromit verwendet werden.

Das Lösungsmittel in Schritt c1) bzw. e2) kann entweder das Keton oder Aldehyd sein, wenn dieses in flüssiger Form vorliegt, oder ein inertes Lösungsmittel, wie Toluol oder Xylol sein, insbesondere wenn das Keton oder Aldehyd in fester Form vorliegt. Im letzteren Fall wird das Keton oder Aldehyd nur in stöchiometrischen Mengen als Reaktant eingesetzt.

Bevorzugt wird ein Keton oder Aldehyd, besonders bevorzugt Keton, in flüssiger Form als Lösungsmittel verwendet. Hierdurch kann auf eine zusätzliche Substanz, wie Toluol oder Xylol, verzichtet werden.

Das Reaktionsprodukt ist, insbesondere nach Schritt c), ein Substanzgemisch umfassend die Verbindung gemäß Formel I), wobei bevorzugt im Anschluss an Schritt c1) bzw. e2) eine Aufreinigung erfolgt, wie beispielsweise säulenchromatographisch, beispielsweise an Kieselgel.

Ein weiterer Gegenstand der Erfindung ist wie oben ausgeführt eine Kautschukmischung.

Die erfindungsgemäße Kautschukmischung enthält die Verbindung gemäß Formel I), insbesondere gemäß Formel II). Bei der erfindungsgemäßen Kautschukmischung kann es sich prinzipiell um jede Kautschukmischung handeln, insbesondere in der die neuartige erfindungsgemäße Verbindung gemäß Formel I), insbesondere gemäß Formel II), als Alterungsschutzmittel und/oder Ozonschutzmittel bei geringerer Toxizität wirkt.

Die erfindungsgemäße Kautschukmischung enthält wenigstens einen Kautschuk.

Bevorzugt enthält die erfindungsgemäße Kautschukmischung 0,1 bis 10 phr, besonders bevorzugt 0,1 bis 7 phr, ganz besonders bevorzugt 1 bis 6 phr, der Verbindung gemäß Formel I), insbesondere gemäß Formel II).

Die in dieser Schrift verwendete Angabe phr (parts per hundred parts of rubber by weight) ist die in der Kautschukindustrie übliche Mengenangabe für Mischungsrezepturen. Die Dosierung der Gewichtsteile der einzelnen Substanzen wird in dieser Schrift auf 100 Gewichtsteile der gesamten Masse aller in der Mischung vorhandenen hochmolekularen (M_{w} größer als 20.000 g/mol) Kautschuke bezogen.

Gemäß vorteilhafter Ausführungsformen der Erfindung enthält die erfindungsgemäße Kautschukmischung wenigstens einen Dienkautschuk. Die Kautschukmischung kann somit einen Dienkautschuk oder ein Gemisch von zwei oder mehreren verschiedenen Dienkautschuken enthalten.

Als Dienkautschuke werden Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Der Dienkautschuk ist dabei bevorzugt ausgewählt aus der Gruppe bestehend aus natürlichem Polyisopren (NR), synthetischem Polyisopren (IR), epoxidiertem Polyisopren (ENR), Butadien-Kautschuk (BR), Butadien-Isopren-Kautschuk, lösungspolymerisiertem Styrol-Butadien-Kautschuk (SSBR), emulsionspolymerisiertem Styrol-Butadien-Kautschuk (ESBR), Styrol-Isopren-Kautschuk, Flüssigkautschuken mit einem Molekulargewicht M_{w} von größer als 20000 g/mol, Halobutyl-Kautschuk, Polynorbornen, Isopren-Isobutylen-Copolymer, Ethylen-Propylen-Dien-Kautschuk, Nitril-Kautschuk, Chloropren-Kautschuk, Acrylat-Kautschuk, Fluor-Kautschuk, Silikon-Kautschuk, Polysulfid-Kautschuk, Epichlorhydrin-Kautschuk, Styrol-Isopren-Butadien-Terpolymer, hydriertem Acrylnitrilbutadien-Kautschuk und hydriertem Styrol-Butadien-Kautschuk.

Insbesondere Nitrilkautschuk, hydrierter Acrylnitrilbutadienkautschuk, Chloroprenkautschuk, Butylkautschuk, Halobutylkautschuk und/oder Ethylen-Propylen-Dien-Kautschuk kommen bei der Herstellung von technischen Gummiartikeln, wie Gurte, Riemen und Schläuche, und/oder Schuhsohlen zum Einsatz. Dabei finden die dem Fachmann für diese Kautschuke bekannten - im Hinblick auf Füllstoffe, Weichmacher, Vulkanisationssysteme und Zuschlagstoffe besonderen - Mischungszusammensetzungen bevorzugte Anwendung.

Bei dem natürlichen und/oder synthetischen Polyisopren sämtlicher Ausführungsformen kann es sich sowohl um cis-1,4-Polyisopren als auch um 3,4-Polyisopren handeln. Bevorzugt ist allerdings die Verwendung von cis-1,4-Polyisoprenen mit einem cis-1,4 Anteil > 90 Gew.-%. Zum einen kann solch ein Polyisopren durch stereospezifische Polymerisation in Lösung mit Ziegler-Natta-Katalysatoren oder unter Verwendung von fein verteilten Lithiumalkylen erhalten werden. Zum anderen handelt es sich bei Naturkautschuk (NR) um ein solches cis-1,4 Polyisopren, bei welchem der cis-1,4-Anteil im Naturkautschuk größer 99 Gew.-% ist.

Ferner ist auch ein Gemisch eines oder mehrerer natürlicher Polyisoprene mit einem oder mehreren synthetischen Polyisopren(en) denkbar.

Im Rahmen der vorliegenden Erfindung ist unter dem Begriff "Naturkautschuk" natürlich vorkommender Kautschuk zu verstehen, der von Hevea Gummibäumen und "Nicht-Hevea" Quellen gewonnen werden kann. Nicht-Hevea Quellen sind beispielsweise Guayule Sträucher und Löwenzahn wie beispielsweise TKS (Taraxacum kok-saghyz; Russischer Löwenzahn).

Falls in der erfindungsgemäßen Kautschukmischung Butadien-Kautschuk (= BR, Polybutadien) enthalten ist, kann es sich um alle dem Fachmann bekannten Typen handeln. Darunter fallen u.a. die sogenannten high-cis- und low-cis-Typen, wobei Polybutadien mit einem cis-Anteil größer oder gleich 90 Gew.-% als high-cis-Typ und Polybutadien mit einem cis-Anteil kleiner als 90 Gew.-% als low-cis-Typ bezeichnet wird. Ein low-cis-Polybutadien ist z.B. Li-BR (Lithium-katalysierter Butadien-Kautschuk) mit einem cis-Anteil von 20 bis 50 Gew.-%. Mit einem high-cis BR werden besonders gute Eigenschaften sowie eine niedrige Hysterese der Kautschukmischung erzielt.

Das oder die eingesetzte(n) Polybutadiene kann/können mit Modifizierungen und Funktionalisierungen endgruppenmodifiziert und/oder entlang der Polymerketten funktionalisiert sein. Bei der Modifizierung kann es sich um solche mit Hydroxy-Gruppen und/oder Ethoxy-Gruppen und/oder Epoxy-Gruppen und/oder Siloxan-Gruppen und/oder Amino-Gruppen und/oder Aminosiloxan und/oder Carboxy-Gruppen und/oder Phthalocyanin-Gruppen und/oder Silan-Sulfid-Gruppen handeln. Es kommen aber auch weitere, dem Fachmann bekannte, Modifizierungen, auch als Funktionalisierungen bezeichnet, in Frage. Bestandteil solcher Funktionalisierungen können Metallatome sein.

Für den Fall, dass wenigstens ein Styrol-Butadien-Kautschuk (Styrol-Butadien-Copolymer) in der Kautschukmischung enthalten ist, kann es sich sowohl um lösungspolymerisierten Styrol-Butadien-Kautschuk (SSBR) als auch um emulsionspolymerisierten Styrol-Butadien-Kautschuk (ESBR) handeln, wobei auch ein Gemisch aus wenigstens einem SSBR und wenigstens einem ESBR eingesetzt werden kann. Die Begriffe "Styrol-Butadien-Kautschuk" und "Styrol-Butadien-Copolymer" werden im Rahmen der vorliegenden Erfindung synonym verwendet.

Das eingesetzte Styrol-Butadien-Copolymer kann mit den oben beim Polybutadien genannten Modifizierungen und Funktionalisierungen endgruppenmodifiziert und/oder entlang der Polymerketten funktionalisiert sein.

Vorzugsweise ist der wenigstens eine Dienkautschuk ausgewählt aus der Gruppe bestehend aus natürlichem Polyisopren (NR, Naturkautschuk), synthetischem Polyisopren (IR), Butadien-Kautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

Gemäß einer besonders bevorzugten Ausführungsform der Erfindung ist der wenigstens eine Dienkautschuk ausgewählt aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer besonders vorteilhaften Ausführungsform der Erfindung enthält die Kautschukmischung wenigstens ein natürliches Polyisopren (NR) und/oder synthetisches Polyisopren (IR) und zwar bevorzugt in Mengen von 50 bis 100 phr, und gemäß einer besonders vorteilhaften Ausführungsform der Erfindung 80 bis 100 phr, ganz besonders bevorzugt 95 bis 100 phr, wiederum bevorzugt 100 phr. Eine derartige Kautschukmischung zeigt insbesondere optimierte Reißeigenschaften und Abriebeigenschaften bei einer guten Verarbeitbarkeit und Reversionsstabilität.

Für den Fall, dass die Kautschukmischung weniger als 100 phr NR und/oder IR enthält, enthält sie bevorzugt als weiteren Kautschuk wenigstens einen Dienkautschuk, der ausgewählt ist aus der Gruppe bestehend aus Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer weiteren besonders vorteilhaften Ausführungsform der Erfindung enthält die Kautschukmischung wenigstens ein natürliches Polyisopren (NR) und zwar bevorzugt in Mengen von 5 bis 55 phr, und gemäß einer besonders vorteilhaften Ausführungsform der Erfindung 5 bis 25 phr, ganz besonders bevorzugt 5 bis 20 phr. Eine derartige Kautschukmischung zeigt insbesondere eine gute Verarbeitbarkeit und Reversionsstabilität sowie optimierte Reißeigenschaften und ein optimales Rollwiderstandsverhalten.

Gemäß einer weiteren besonders vorteilhaften Ausführungsform der Erfindung enthält die Kautschukmischung wenigstens ein Polybutadien (BR, Butadienkautschuk) und zwar bevorzugt in Mengen von 10 bis 80 phr, besonders bevorzugt 10 bis 50 phr, und gemäß einer besonders vorteilhaften Ausführungsform der Erfindung 15 bis 40 phr. Hiermit werden besonders gute Reiß- und Abriebeigenschaften der erfindungsgemäßen Kautschukmischung und ein optimales Bremsverhalten erzielt.

Gemäß einer weiteren besonders vorteilhaften Ausführungsform der Erfindung enthält die Kautschukmischung wenigstens einen lösungspolymerisierten Styrol-Butadien-Kautschuk (SSBR) und zwar bevorzugt in Mengen von 10 bis 80 phr, besonders bevorzugt 30 bis 80 phr, und gemäß einer besonders vorteilhaften Ausführungsform der Erfindung 50 bis 70 phr. Hiermit werden besonders gute Rollwiderstandseigenschaften der erfindungsgemäßen Kautschukmischung erzielt. Gemäß besonders vorteilhaften Ausführungsformen der Erfindung wird SSBR in Kombination mit wenigstens einem weiteren Kautschuk eingesetzt, um ein optimales und ausbalanciertes Eigenschaftsprofil zu erzielen.

Bevorzugt enthält die Kautschukmischung wenigstens einen Füllstoff, bevorzugt in Mengen von 30 bis 500 phr, besonders bevorzugt 50 bis 400 phr, wiederum bevorzugt 80 bis 300 phr.

Gemäß vorteilhafter Ausführungsformen der Erfindung ist der Füllstoff ein verstärkender Füllstoff, der bevorzugt ausgewählt ist aus der Gruppe bestehend aus Rußen und Siliciumdioxid.

Als Ruße kommen alle dem Fachmann bekannten Rußtypen in Frage. Bevorzugt ist der Ruß ausgewählt aus Industrierußen und Pyrolyse-Rußen, wobei Industrieruße weiter bevorzugt sind.

Bevorzugt hat der Ruß eine Jodzahl, gemäß ASTM D 1510, die auch als Jodadsorptionszahl bezeichnet wird, zwischen 30 und 250 g/kg, bevorzugt 30 bis 180 g/kg, besonders bevorzugt 40 bis 180 g/kg, und ganz besonders bevorzugt 40 bis 130 g/kg, und eine DBP-Zahl gemäß ASTM D 2414 von 30 bis 200 ml/100 g, bevorzugt 70 bis 200 ml/100g, besonders bevorzugt 90 bis 200 ml/100g.

Die DBP-Zahl gemäß ASTM D 2414 bestimmt das spezifische Absorptionsvolumen eines Rußes oder eines hellen Füllstoffes mittels Dibutylphthalat.

Die Verwendung eines solchen Rußtyps in der Kautschukmischung, insbesondere für Fahrzeugreifen, gewährleistet einen bestmöglichen Kompromiss aus Abriebwiderstand und Wärmeaufbau, der wiederum den ökologisch relevanten Rollwiderstand beeinflusst.

Besonders geeignet und bevorzugt ist dabei ein Ruß mit einer Jodadsorptionszahl zwischen 80 und 110 g/kg und einer DBP-Zahl von 100 bis 130 ml/100g, wie insbesondere Rußes des Types N339.

Das Siliciumdioxid ist bevorzugt amorphes Siliciumdioxid, beispielsweise gefällte Kieselsäure, die auch als gefälltes Siliciumdioxid bezeichnet wird. Alternativ kann aber beispielsweise auch pyrogenes Siliciumdioxid eingesetzt werden. Besonders bevorzugt ist es allerdings, wenn eine fein verteilte, gefällte Kieselsäure verwendet wird, die eine Stickstoff-Oberfläche (BET-Oberfläche) (gemäß DIN ISO 9277 und DIN 66132) von 35 bis 400 m²/g, bevorzugt von 35 bis 350 m²/g, besonders bevorzugt von 85 bis 320 m²/g und ganz besonders bevorzugt von 120 bis 235 m²/g, und eine CTAB-Oberfläche (gemäß ASTM D 3765) von 30 bis 400 m²/g, bevorzugt von 30 bis 330 m²/g, besonders bevorzugt von 80 bis 300 m²/g und ganz besonders bevorzugt von 115 bis 200 m²/g, aufweist. Derartige Kieselsäuren führen z. B. in Kautschukmischungen für Reifenlaufstreifen zu besonders guten physikalischen Eigenschaften der Vulkanisate. Außerdem können sich dabei Vorteile in der Mischungsverarbeitung durch eine Verringerung der Mischzeit bei gleichbleibenden Produkteigenschaften ergeben, die zu einer verbesserten Produktivität führen. Als Kieselsäuren können somit z. B. sowohl jene des Typs Ultrasil^{®} VN3 (Handelsname) der Firma Evonik als auch hoch dispergierbare Kieselsäuren, so genannte HD-Kieselsäuren (z. B. Zeosil^{®} 1165 MP der Firma Solvay), zum Einsatz kommen.

Gemäß besonders vorteilhafter Ausführungsformen der Erfindung enthält die Kautschukmischung wenigstens eine Kieselsäure als Füllstoff, bevorzugt in Mengen von 30 bis 500 phr, besonders bevorzugt 50 bis 400 phr, wiederum bevorzugt 80 bis 300 phr.

In diesen Mengen ist Kieselsäure insbesondere als alleiniger oder als Hauptfüllstoff (mehr als 50 Gew.-% bezogen auf die Gesamtfüllstoffmenge) enthalten.

Gemäß weiterer vorteilhafter Ausführungsformen der Erfindung enthält die Kautschukmischung wenigstens eine Kieselsäure als weiteren Füllstoff, und zwar bevorzugt in Mengen von 5 bis 100 phr, besonders bevorzugt 5 bis 80 phr, wiederum bevorzugt 10 bis 60 phr.

In diesen Mengen ist Kieselsäure insbesondere als weiterer Füllstoff zusätzlich zu einem anderen Hauptfüllstoff, wie insbesondere einem Ruß, enthalten.

Die Begriffe "Kieselsäure" und "Silika" werden im Rahmen der vorliegenden Erfindung synonym verwendet.

Gemäß besonders vorteilhafter Ausführungsformen der Erfindung enthält die erfindungsgemäße Kautschukmischung 0,1 bis 60 phr, bevorzugt 3 bis 40 phr, besonders bevorzugt 5 bis 30 phr, ganz besonders bevorzugt 5 bis 15 phr, wenigstens eines Rußes. In diesen Mengen ist Ruß insbesondere als weiterer Füllstoff zusätzlich zu einem Hauptfüllstoff, wie insbesondere Kieselsäure, enthalten.

Gemäß weiterer vorteilhafter Ausführungsformen der Erfindung enthält die erfindungsgemäße Kautschukmischung 30 bis 300 phr, bevorzugt 30 bis 200 phr, besonders bevorzugt 40 bis 100 phr wenigstens eines Rußes. In diesen Mengen ist Ruß als alleiniger oder als Hauptfüllstoff und dabei ggf. in Kombination mit Kieselsäure in den oben genannten geringeren Mengen enthalten.

Gemäß einer besonders vorteilhaften Ausführungsform der Erfindung enthält die Kautschukmischung 5 bis 60 phr, besonders bevorzugt 5 bis 40 phr, wenigstens eines Rußes und 50 bis 300 phr, bevorzugt 80 bis 200 phr wenigstens einer Kieselsäure.

Die Kautschukmischung kann zudem weitere Füllstoffe enthalten, die verstärkend wirken oder nicht verstärkend wirken.

Zu den weiteren (nicht verstärkenden) Füllstoffen zählen im Rahmen der vorliegenden Erfindung Alumosilicate, Kaolin, Kreide, Stärke, Magnesiumoxid, Titandioxid oder Kautschukgele sowie Fasern (wie zum Beispiel Aramidfasern, Glasfasern, Carbonfasern, Cellulosefasern).

Weitere ggf. verstärkende Füllstoffe sind z.B. Kohlenstoffnanoröhrchen (carbon nanotubes (CNT) inklusive diskreter CNTs, sogenannte hollow carbon fibers (HCF) und modifizierte CNT enthaltend eine oder mehrere funktionelle Gruppen, wie Hydroxy-, Carboxy und Carbonyl-Gruppen), Graphit und Graphene und sogenannte "carbon-silica dual-phase filler".

Zinkoxid gehört im Rahmen der vorliegenden Erfindung nicht zu den Füllstoffen.

Des Weiteren kann die Kautschukmischung übliche Zusatzstoffe in üblichen Gewichtsteilen enthalten, die bei deren Herstellung bevorzugt in wenigstens einer Grundmischstufe zugegeben werden. Zu diesen Zusatzstoffen zählen
a) im Stand der Technik bekannte Alterungsschutzmittel,
   wie z. B. p-Phenylendiamine, wie
   N-Phenyl-N'-(1,3-dimethylbutyl)-p-phenylendiamin (6PPD),
   N,N'-Diphenyl-p-phenylendiamin (DPPD),
   N-(1-phenylethyl)-N'-phenyl-p-phenylendiamin (SPPD),
   N,N'-Ditolyl-p-phenylendiamin (DTPD),
   N-(1,4-dimethylpentyl)-N'-phenyl-p-phenylendiamin (7PPD),
   N-Isopropyl-N'-phenyl-p-phenylendiamin (IPPD),
   oder Dihydrochinoline, wie 2,2,4-Trimethyl-1,2-dihydrochinolin (TMQ),
b) Aktivatoren, wie z. B. Zinkoxid und Fettsäuren (z. B. Stearinsäure) und/oder sonstige Aktivatoren, wie Zinkkomplexe wie z.B. Zinkethylhexanoat,
c) Aktivatoren und/oder Agenzien für die Anbindung von Füllstoffen, insbesondere Ruß oder Kieselsäure, wie beispielsweise S-(3-Aminopropyl)Thioschwefelsäure und/oder deren Metallsalze (Anbindung an Ruß) sowie Silan-Kupplungsagenzien (Anbindung an Siliciumdioxid, insbesondere Kieselsäure),
d) Ozonschutzwachse,
e) Harze, insbesondere Klebharze,
f) Mastikationshilfsmittel, wie z. B. 2,2'-Dibenzamidodiphenyldisulfid (DBD) und
g) Prozesshilfsmittel, wie insbesondere Fettsäureester und Metallseifen, wie z.B. Zinkseifen und/oder Calciumseifen
h) Weichmacher, wie insbesondere wie aromatische, naphthenische oder paraffinische Mineralölweichmacher, wie z.B. MES (mild extraction solvate) oder RAE (Residual Aromatic Extract) oder TDAE (treated distillate aromatic extract), oder Rubber-to-Liquid-Öle (RTL) oder Biomass-to-Liquid-Öle (BTL) bevorzugt mit einem Gehalt an polycyclischen Aromaten von weniger als 3 Gew.-% gemäß Methode IP 346 oder Triglyceride, wie z. B. Rapsöl, oder Faktisse oder Kohlenwasserstoffharze oder Flüssig-Polymere, deren mittleres Molekulargewicht (Bestimmung per GPC = gel permeation chromatography, in Anlehnung an BS ISO 11344:2004) zwischen 500 und 20000 g/mol liegt.

Bei der Verwendung von Mineralöl ist dieses bevorzugt ausgewählt aus der Gruppe, bestehend aus DAE (Destillated Aromatic Extracts), RAE (Residual Aromatic Extract), TDAE (Treated Destillated Aromatic Extracts), MES (Mild Extracted Solvents) und naphthenischen Ölen.

Gemäß besonders vorteilhafter Ausführungsformen enthält die erfindungsgemäße Kautschukmischung neben der erfindungsgemäßen Verbindung gemäß Formel I), insbesondere gemäß Formel II), keine Alterungsschutzmittel aus der Gruppe der p-Phenylendiamine, insbesondere solche unter obiger Auflistung a). Insbesondere enthält die erfindungsgemäße Kautschukmischung gemäß einer besonders bevorzugten Ausführungsform 0 bis 0,1 phr, insbesondere 0 phr, an weiteren Alterungsschutzmitteln auf Basis von p-Phenylendiaminen, die ausgewählt sind aus der Gruppe enthaltend, bevorzugt bestehend aus,
N-Phenyl-N'-(1,3-dimethylbutyl)-p-phenylendiamin (6PPD),
N-(1-phenylethyl)-N'-phenyl-p-phenylendiamin (SPPD),
N,N'-Diphenyl-p-phenylendiamin (DPPD), N,N'-Ditolyl-p-phenylendiamin (DTPD),
N-Isopropyl-N'-phenyl-p-phenylendiamin (IPPD),
N-(1,4-dimethylpentyl)-N'-phenyl-p-phenylendiamin (7PPD).

Mit den bevorzugt sehr geringen Mengen von 0 bis 0,1 phr bzw. besonders bevorzugt 0 phr an p-Phenylendiaminen, und der erfindungsgemäß enthaltenen Verbindung gemäß Formel I), insbesondere gemäß Formel II), ist es möglich eine verbesserte Schutzwirkung bei geringerer Toxizität zu erzielen. Hierbei ersetzt die erfindungsgemäße Verbindung gemäß Formel I), insbesondere gemäß Formel II), die genannten im Stand der Technik bekannten p-Phenylendiamine.

Gemäß weiterer vorteilhafter Ausführungsformen der Erfindung ist noch wenigstens ein weiteres der genannten p-Phenylendiamin-Alterungsschutzmittel enthalten, sodass die erfindungsgemäße Verbindung die im Stand der Technik bekannten p-Phenylendiamine nur teilweise ersetzt. Hierdurch wird der erfindungsgemäße Vorteil auch erzielt, nur nicht in optimalem Ausmaß.

Alterungsschutzmittel auf Basis von Dihydrochinolin, wie TMQ, sind gemäß vorteilhafter Ausführungsformen neben der erfindungsgemäßen Verbindung gemäß Formel I) in der Kautschukmischung enthalten. Die Menge an enthaltenen Dihydrochinolinen, wie insbesondere TMQ, beträgt bevorzugt 0,1 bis 3, insbesondere 0,5 bis 1,5 phr.

Ozonschutzwachse (obige Gruppe d) werden separat betrachtet und sind gemäß bevorzugter Ausführungsformen der Erfindung in der Kautschukmischung enthalten, unabhängig davon ob zusätzliche Alterungsschutzmittel a) enthalten sind.

Bei den Silan-Kupplungsagenzien kann es sich um alle dem Fachmann bekannten Typen handeln.

Ferner können ein oder mehrere verschiedene Silan-Kupplungsagenzien in Kombination miteinander eingesetzt werden. Die Kautschukmischung kann somit ein Gemisch verschiedener Silane enthalten.

Die Silan-Kupplungsagenzien reagieren mit den oberflächlichen Silanolgruppen des Siliciumdioxids, insbesondere der Kieselsäure, oder anderen polaren Gruppen während des Mischens des Kautschuks bzw. der Kautschukmischung (in situ) oder bereits vor der Zugabe des Füllstoffes zum Kautschuk im Sinne einer Vorbehandlung (Vormodifizierung).

Aus dem Stand der Technik bekannten Kupplungsagenzien sind bifunktionelle Organosilane, die am Siliciumatom mindestens eine Alkoxy-, Cycloalkoxy- oder Phenoxygruppe als Abgangsgruppe besitzen und die als andere Funktionalität eine Gruppe aufweisen, die gegebenenfalls nach Spaltung eine chemische Reaktion mit den Doppelbindungen des Polymers eingehen kann. Bei der letztgenannten Gruppe kann es sich z. B. um die folgenden chemischen Gruppen handeln: -SCN, -SH, -NH₂ oder -Sₓ- (mit x = 2 bis 8).

So können als Silan-Kupplungsagenzien z. B. 3-Mercaptopropyltriethoxysilan, 3-Thiocyanato-propyltrimethoxysilan oder 3,3'-Bis(triethoxysilylpropyl)polysulfide mit 2 bis 8 Schwefelatomen, wie z. B. 3,3'-Bis(triethoxysilylpropyl)tetrasulfid (TESPT), das entsprechende Disulfid (TESPD) oder auch Gemische aus den Sulfiden mit 1 bis 8 Schwefelatomen mit unterschiedlichen Gehalten an den verschiedenen Sulfiden, verwendet werden. TESPT kann dabei beispielsweise auch als Gemisch mit Industrieruß (Handelsname X50S^{®} der Firma Evonik) zugesetzt werden.

Auch geblockte Mercaptosilane, wie sie z. B. aus der WO 99/09036 bekannt sind, können als Silan-Kupplungsagens eingesetzt werden. Auch Silane, wie sie in der WO 2008/083241 A1, der WO 2008/083242 A1, der WO 2008/083243 A1 und der WO 2008/083244 A1 beschrieben sind, können eingesetzt werden. Verwendbar sind z. B. Silane, die unter dem Namen NXT in verschiedenen Varianten von der Firma Momentive, USA, wie insbesondere 3-Octanoylthio-1-propyltriethoxysilan, oder solche, die unter dem Namen VP Si 363^{®} von der Firma Evonik Industries vertrieben werden.

Der Mengenanteil der Gesamtmenge an weiteren Zusatzstoffen beträgt bevorzugt 3 bis 150 phr, besonders bevorzugt 3 bis 100 phr und ganz besonders bevorzugt 5 bis 80 phr.

Im Gesamtmengenanteil der weiteren Zusatzstoffe kann Zinkoxid (ZnO) in den oben genannten Mengen enthalten sein.

Hierbei kann es sich um alle dem Fachmann bekannten Typen an Zinkoxid handeln, wie z.B. ZnO-Granulat oder -Pulver. Das herkömmlicherweise verwendete Zinkoxid weist in der Regel eine BET-Oberfläche von weniger als 10 m²/g auf. Es kann aber auch ein Zinkoxid mit einer BET-Oberfläche von 10 bis 100 m²/g, wie z.B. so genannte "nano-Zinkoxide", verwendet werden.

Die erfindungsgemäße Kautschukmischung wird bevorzugt vulkanisiert verwendet, insbesondere in Fahrzeugreifen oder anderen vulkanisierten technischen Gummiartikeln.

Die Begriffe "vulkanisiert" und "vernetzt" werden im Rahmen der vorliegenden Erfindung synonym verwendet.

Die Vulkanisation der erfindungsgemäßen Kautschukmischung wird bevorzugt in Anwesenheit von Schwefel und/oder Schwefelspendern mit Hilfe von Vulkanisationsbeschleunigern durchgeführt, wobei einige Vulkanisationsbeschleuniger zugleich als Schwefelspender wirken können. Dabei ist der Beschleuniger ausgewählt aus der Gruppe bestehend aus Thiazolbeschleunigern, Mercaptobeschleunigern, Sulfenamidbeschleunigern, Thiocarbamatbeschleunigern, Thiurambeschleunigern, Thiophosphatbeschleunigern, Thioharnstoffbeschleunigern, Xanthogenat-Beschleunigern und Guanidin-Beschleunigern. Bevorzugt ist die Verwendung eines Sulfenamidbeschleunigers, der ausgewählt ist aus der Gruppe bestehend aus N-Cyclohexyl-2-benzothiazolsufenamid (CBS), N,N-Dicyclohexylbenzothiazol-2-sulfenamid (DCBS), Benzothiazyl-2-sulfenmorpholid (MBS), N-tert-Butyl-2-benzothiazylsulfenamid (TBBS) und Guanidin-Beschleunigern wie Diphenylguanidin (DPG).

Als schwefelspendende Substanz können dabei alle dem Fachmann bekannten schwefelspendenden Substanzen verwendet werden.

Außerdem können in der Kautschukmischung Vulkanisationsverzögerer vorhanden sein.

Die Herstellung der Kautschukmischung erfolgt bevorzugt ansonsten nach dem in der Kautschukindustrie üblichen Verfahren, bei dem zunächst in ein oder mehreren Mischstufen eine Grundmischung mit allen Bestandteilen außer dem Vulkanisationssystem (z. B. Schwefel und vulkanisationsbeeinflussende Substanzen) hergestellt wird. Durch Zugabe des Vulkanisationssystems in einer letzten Mischstufe wird die Fertigmischung erzeugt.

Die Fertigmischung wird z.B. durch einen Extrusionsvorgang oder Kalandrieren weiterverarbeitet und in die entsprechende Form gebracht.

Die erfindungsgemäße Kautschukmischung ist besonders für die Verwendung in Fahrzeugreifen, insbesondere Fahrzeugluftreifen geeignet. Hierbei ist die Anwendung in allen Reifenbauteilen prinzipiell denkbar, insbesondere in einem äußeren Bauteil, insbesondere und bevorzugt im Hornprofil, Laufstreifen und/oder der Seitenwand Laufstreifen. Im Falle eines Laufstreifens mit Cap/Base-Konstruktion wird die erfindungsgemäße Kautschukmischung bevorzugt wenigstens in der Cap verwendet.

Zur Verwendung in Fahrzeugreifen wird die Mischung als Fertigmischung vor der Vulkanisation in die entsprechende Form, bevorzugt eines äußeren Bauteils, gebracht und bei der Herstellung des Fahrzeugreifenrohlings wie bekannt aufgebracht.

Die Herstellung der erfindungsgemäßen Kautschukmischung zur Verwendung als sonstige Body-Mischung in Fahrzeugreifen erfolgt wie bereits beschrieben. Der Unterschied liegt in der Formgebung nach dem Extrusionsvorgang bzw. dem Kalandrieren der Mischung. Die so erhaltenen Formen der noch unvulkanisierten Kautschukmischung für eine oder mehrere unterschiedliche Body-Mischungen dienen dann dem Aufbau eines Reifenrohlings.

Als Body-Mischung werden hierbei die Kautschukmischungen für die inneren Bauteile eines Reifen bezeichnet, wie im Wesentlichen Squeegee, Innenseele (Innenschicht), Kernprofil, Gürtel, Schulter, Gürtelprofil, Karkasse, Wulstverstärker, Wulstprofil, Hornprofil und Bandage.

Der noch unvulkanisierte Reifenrohling wird anschließend vulkanisiert.

Zur Verwendung der erfindungsgemäßen Kautschukmischung in Riemen und Gurten, insbesondere in Fördergurten, wird die extrudierte noch unvulkanisierte Mischung in die entsprechende Form gebracht und dabei oder nachher häufig mit Festigkeitsträgern, z.B. synthetische Fasern oder Stahlcorde, versehen. Zumeist ergibt sich so ein mehrlagiger Aufbau, bestehend aus einer und/oder mehrerer Lagen Kautschukmischung, einer und/oder mehrerer Lagen gleicher und/oder verschiedener Festigkeitsträger und einer und/oder mehreren weiteren Lagen dergleichen und/oder einer anderen Kautschukmischung.

Ein weiterer Gegenstand der vorliegenden Erfindung ist ein Fahrzeugreifen, der die erfindungsgemäße Kautschukmischung enthaltend die erfindungsgemäße Verbindung in wenigstens einem Bauteil aufweist.

Der vulkanisierte Fahrzeugreifen weist wenigstens in einem Bauteil ein Vulkanisat wenigstens einer erfindungsgemäßen Kautschukmischung auf. Dem Fachmann ist bekannt, dass die meisten Substanzen, wie z. B. die enthaltenen Kautschuke entweder bereits nach dem Mischen oder erst nach der Vulkanisation in chemisch veränderter Form vorliegen oder vorliegen können.

Unter Fahrzeugreifen werden im Rahmen der vorliegenden Erfindung Fahrzeugluftreifen und Vollgummireifen, inklusive Reifen für Industrie- und Baustellenfahrzeuge, LKW-, PKW- sowie Zweiradreifen verstanden.

Bevorzugt weist der erfindungsgemäße Fahrzeugreifen die erfindungsgemäße Kautschukmischung in wenigstens einem äußeren Bauteil auf, wobei das äußere Bauteil bevorzugt ein Laufstreifen, eine Seitenwand und/oder ein Hornprofil ist.

Der erfindungsgemäße Fahrzeugreifen kann die erfindungsgemäße Kautschukmischung enthaltend die erfindungsgemäße Verbindung gemäß Formel I), insbesondere gemäß Formel II), somit auch in mehreren Bauteilen in ggf. angepasster Zusammensetzung aufweisen.

Im Folgenden soll die Erfindung anhand von Ausführungsbeispielen näher erläutert werden.

Die Verbindung gemäß Formel II) als bevorzugte Ausführungsform der Verbindung gemäß Formel I) wurde auf folgende Weise gemäß einer ersten Syntheseroute hergestellt:
Zunächst wurde 2-Nitroacridin-9(10H)-on nach R. Freyer J. Chem. 1963, 4979-5004 - wie in Schema YI) dargestellt - synthetisiert: 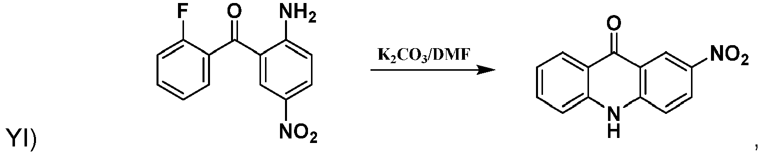 wobei K₂CO₃ für Kaliumcarbonat und DMF für Dimethylformamid stehen.

Hieraus erfolgte die Synthese von 7-((4-Methylpentan-2-yl)amino)-1,3,4,10-tetrahydroacridin-9(2H)-on (Verbindung gemäß Formel II) gemäß Schema YII):

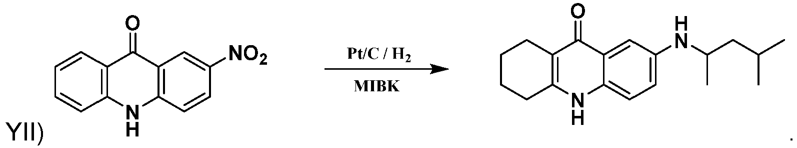

In einen Edelstahlautoklaven, der mit einem Tefloninliner bestückt wurde, wurden 0.55 g (2.62 mmol, 1 Äq) 2-Nitroacridin-9(10H)-on, 0.224 g Platin auf Kohle (5%) (0.4 g auf 4.67 mmol Substrat) und 20.0 mL Methylisobutylketon eingewogen. Anschließend wurde 40 bar Wasserstoff aufgedrückt und bei 140°C für 10 Stunden rühren gelassen. Nach Beendigung der Reaktion wurde der überschüssige Wasserstoff abgeblasen und die Suspension über Celite^{®} filtriert sowie mit Ethanol nachgewaschen. Das Filtrat wurde bis zur Trockne eingeengt und im Vakuum getrocknet. Der Rückstand wurde per LC-MS analysiert. Das Ergebnis ist in Tabelle 1 gezeigt.

**Tabelle 1**

| **Substanz** | **Ausbeute [%]** | **Retentionszeit [min]** |
|---|---|---|
| 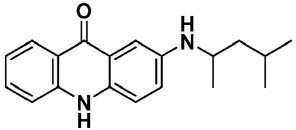 | 45 | 3.86 |
| 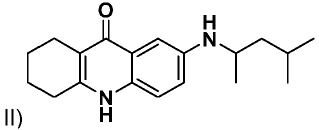 | 44 | 3.56 |
| 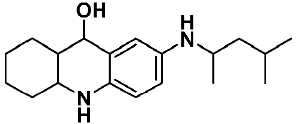 | 10 | 2.6 |

Die Substanz kann säulenchromatographisch an Kieselgel aufgereinigt werden (Cyclohexan/Essigester 10:1 → 1:1). Hellgelber Feststoff; Ausbeute 0.34 g (40 % d. Th.).

### Analytik von 7-((4-Methylpentan-2-yl)amino)-1,3,4,10-tetrahydroacridin-9(2H)-on:

¹H-NMR (*engl.* "nuclear magnetic resonance") (500 MHz, DMSO-*d*₆) δ = 11.02 (s, 1H), 7.23 (d, *J* = 8.9 Hz, 1H), 7.04 (d, *J* = 2.7 Hz, 1H), 6.97 (dd, *J* = 8.9, 2.7 Hz, 1H), 5.35 (d, *J =* 8.4 Hz, 1H), 3.54 - 3.41 (m, 1H), 2.65 (t, *J* = 6.2 Hz, 2H), 2.42 (t, *J* = 6.2 Hz, 2H), 1.85 - 1.60 (m, 5H), 1.48 (dt, *J* = 13.9, 7.1 Hz, 1H), 1.24 (ddd, *J =* 13.6, 8.6, 5.8 Hz, 1H), 1.10 (d, *J* = 6.2 Hz, 3H), 0.93 (d, *J* = 6.6 Hz, 3H), 0.87 (d, *J* = 6.6 Hz, 3H).

¹³C-NMR (126 MHz, DMSO) δ = 175.7, 145.0, 144.3, 131.6, 125.2, 121.0, 118.7, 114.0, 102.3, 46.2, 46.2, 27.5, 26.8, 25.0, 23.2, 23.0, 22.7, 22.4, 22.2, 20.9.

ESI-MS (Elektrosprayionisation Massenspektrometrie) [M+H]⁺ = 299.

Die Verbindung gemäß Formel II) wurde zudem - wie in Schema XI) und XII) dargestellt - gemäß einer weiteren Syntheseroute synthetisiert:

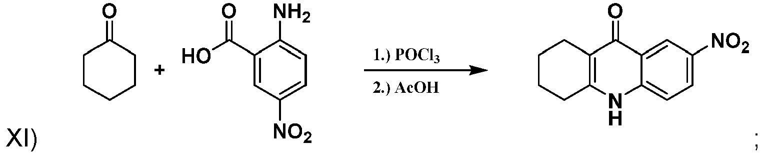

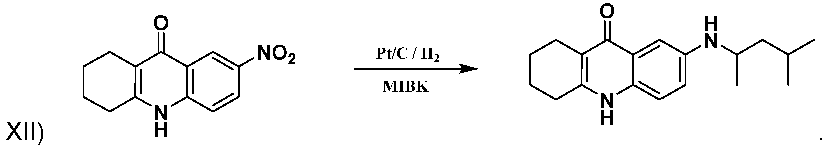

Die Syntheseschritte gemäß Schema XI) folgten dabei der Literatur, s. Cross, R. Matthew et al.; Journal of Medicinal Chemistry (2011), 54(13), 4399-4426.

Die Syntheseschritte gemäß Schema XII) (Synthese von 7-((4-Methylpentan-2-yl)amino)-1,3,4,10-tetrahydroacridin-9(2H)-on, Molekül gemäß Formel II), erfolgte folgendermaßen:
In einen Edelstahlautoklaven, der mit einem Tefloninliner bestückt wurde, wurden 0.24 g (0.98 mmol, 1 Äq) 6-Nitro-1,3,4,10-tetrahydroacridin-9(2H)-on, 0.082 g Platin auf Kohle (5%) (0.4 g auf 4.67 mmol Substrat) und 20.0 mL Methylisobutylketon (MIBK) eingewogen. Anschließend wurde 20 bar Wasserstoff aufgedrückt und bei 60°C für 10 Stunden rühren gelassen. Nach Beendigung der Reaktion wurde der überschüssige Wasserstoff abgeblasen und die Suspension über Celite^{®} filtriert sowie mit Ethanol nachgewaschen. Das Filtrat wurde bis zur Trockne eingeengt und im Vakuum getrocknet; Ausbeute 98 %; Analytik siehe oben.

### Messung der Oxidations-Induktions-Zeit (OIT, engl. "oxidation induction time")

Die Verbindung gemäß Formel II) wurde durch Messung der Oxidations-Induktions-Zeit unter Laborbedingungen auf ihre potenzielle Schutzwirkung als Alterungsschutzmittel untersucht. Hierzu wurden die Verbindungen gemäß Formel II) sowie 6-PPD jeweils zusammen mit einem Polymer (flüssiges synthetisches Polyisopren (IR), LIR-50, Fa. Kuraray, Gewichtsmittel der Molekulargewichtsverteilung M_{w} = 54.000 g/mol, Glasübergangstemperatur T_{g} = -63°C) bei konstanter Temperatur (180°C) erhitzt bis Oxidation eintrat (Starttemperatur 35 °C, Aufheizen auf 170°C mit einer Heizrate von 20 K/min (Kelvin pro Minute), Aufheizen auf 180°C mit einer Heizrate von 1 K/min; Spülgas: Stickstoff (N₂) mit einem Volumenstrom von 50 mL/min).

Die Probe wurde 5 Minuten bei 180°C isotherm unter N₂-Atmosphäre gehalten und anschließend wurde auf O₂-Atmosphäre (mit einem Volumenstrom von 50 mL/min) umgestellt.

Die Oxidation wurde über einen Peak mittels DSC (dynamische Differenzkalorimetrie; *engl.* "differential scanning calorimetry") ermittelt.

Gemessen wurde die Zeit in Minuten bis zur Oxidation.

Die Ergebnisse sind im Vergleich zu dem bekannten Alterungsschutzmittel 6-PPD in Tabelle 2 zusammengefasst.

**Tabelle 2**

| **Substanz** | **Zeit [min]** |
|---|---|
| 6-PPD | 116±10 |
| 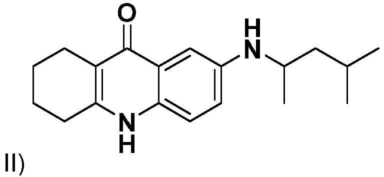 | 212±10 |

Unter Berücksichtigung der Messgenauigkeit von ± (Plusminus) 10 Minuten zeigt sich, dass die Verbindung gemäß Formel II) sogar eine deutlich bessere Schutzwirkung erzielt, da es länger dauert, bis das Polymer durch Sauerstoff zersetzt und damit oxidiert wird. Damit ist die erfindungsgemäße Verbindung gemäß Formel I) bzw. Formel II) umwelt- und gesundheitsfreundlicher als 6-PPD bzw. weitere Vertreter der Substanzklasse, wie eingangs ausgeführt, und zudem ein besseres Alterungsschutzmittel.

## Patentansprüche

1. Verbindung gemäß Formel I): 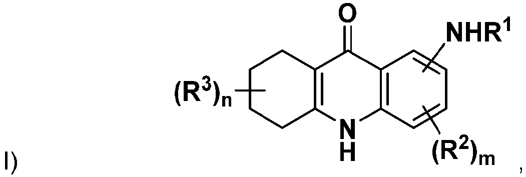
wobei R¹ ausgewählt ist aus der Gruppe bestehend aus Benzyl-Resten, 1-Phenylalkyl-Resten mit insgesamt 7 bis 18 Kohlenstoffatomen und linearen, verzweigten und cyclischen aliphatischen C₃- bis C₁₂-Resten; und
wobei R³ ausgewählt ist aus der Gruppe bestehend aus linearen, verzweigten und cyclischen aliphatischen C₁- bis C₁₂-Resten, sowie Aryl-Resten, Ether-Resten und Thioether-Resten, und wobei n den Wert 0 oder 1 oder 2 oder 3 oder 4 annimmt, wobei die Reste R³ im Fall von n gleich 2 oder 3 oder 4 unabhängig voneinander gleich oder verschieden sind, und
wobei R² ausgewählt ist aus der Gruppe bestehend aus linearen, verzweigten und cyclischen aliphatischen C₁- bis C₁₂-Resten, sowie Aryl-Resten, Ether-Resten und Thioether-Resten; und
wobei m den Wert 0 oder 1 oder 2 oder 3 annimmt, wobei die Reste R² im Fall von m gleich 2 oder 3 unabhängig voneinander gleich oder verschieden sind.

2. Verbindung nach Anspruch 1, **dadurch gekennzeichnet, dass** n gleich 0 (null) ist.

3. Verbindung nach Anspruch 1 oder 2, **dadurch gekennzeichnet, dass** m gleich 0 (null) ist.

4. Verbindung nach einem der vorherigen Ansprüche, **dadurch gekennzeichnet, dass** R¹ über ein tertiäres Kohlenstoffatom an das Stickstoffatom (N) gebunden ist.

5. Verbindung nach einem der vorherigen Ansprüche, **dadurch gekennzeichnet, dass** R¹ ein verzweigter Alkyl-Rest mit 3 bis 12 Kohlenstoffatomen, bevorzugt 4 bis 8 Kohlenstoffatomen, oder ein 1-Phenylalkyl-Rest mit insgesamt 7 bis 10 Kohlenstoffatomen ist.

6. Verbindung nach einem der vorherigen Ansprüche, **dadurch gekennzeichnet, dass** R¹ ausgewählt ist aus der Gruppe bestehend aus 1,3-Dimethylbutyl-, 1-Phenylethyl- und Cyclohexyl-Resten, wobei R¹ bevorzugt ein 1,3-Dimethylbutyl-Rest ist.

7. Verbindung nach einem der vorherigen Ansprüche, **dadurch gekennzeichnet, dass** sie die Struktur gemäß Formel II) aufweist:

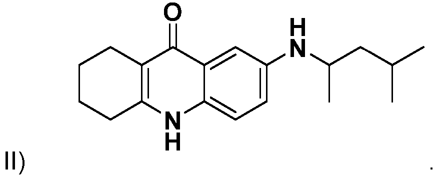

8. Verwendung der Verbindung nach einem der Ansprüche 1 bis 7 als Alterungsschutzmittel, insbesondere in Fahrzeugreifen oder technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen.

9. Verwendung der Verbindung nach einem der Ansprüche 1 bis 7 als Farbstoff in Fasern und/oder Polymeren und/oder Papier und/oder in (Streich-) Farben und Lacken.

10. Verfahren zur Herstellung der Verbindung gemäß Formel I), welches die folgenden Verfahrensschritte umfasst:
a1) Bereitstellung der Verbindung gemäß Formel A1) 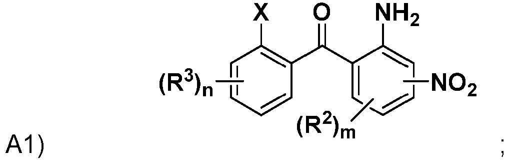
b1) Umsetzung der Verbindung gemäß Formel A1) mit einer Base, insbesondere Kaliumcarbonat (K₂CO₃), wobei die Verbindung gemäß Formel B1) erhalten wird: 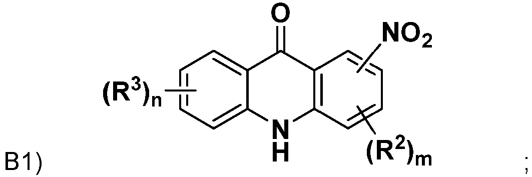
c1) Umsetzung der Verbindung gemäß Formel B1) mit Wasserstoff oder einem Hydrierungsreagenz, und einem Keton oder Aldehyd, zu der Verbindung gemäß Formel I): 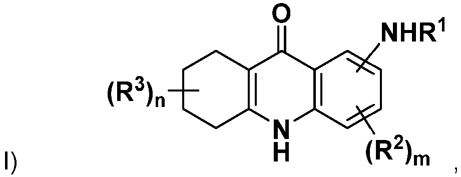 wobei R¹ ausgewählt ist aus der Gruppe bestehend aus Benzyl-Resten, 1-Phenylalkyl-Resten mit insgesamt 7 bis 18 Kohlenstoffatomen und linearen, verzweigten und cyclischen aliphatischen C₃- bis C₁₂-Resten; und wobei R³ ausgewählt ist aus der Gruppe bestehend aus linearen, verzweigten und cyclischen aliphatischen C₁- bis C₁₂-Resten, sowie Aryl-Resten, Ether-Resten und Thioether-Resten, und wobei n den Wert 0 oder 1 oder 2 oder 3 oder 4 annimmt, wobei die Reste R³ im Fall von n gleich 2 oder 3 oder 4 unabhängig voneinander gleich oder verschieden sind, und wobei R² ausgewählt ist aus der Gruppe bestehend aus linearen, verzweigten und cyclischen aliphatischen C₁- bis C₁₂-Resten, sowie Aryl-Resten, Ether-Resten und Thioether-Resten; und wobei m den Wert 0 oder 1 oder 2 oder 3 annimmt, wobei die Reste R² im Fall von m gleich 2 oder 3 unabhängig voneinander gleich oder verschieden sind, und wobei X ein Halogen, insbesondere Fluor (F), Chlor (Cl) oder Brom (Br), ist.

11. Verfahren nach Anspruch 10, **dadurch gekennzeichnet, dass** die Umsetzung in Schritt c1) mit Wasserstoff und dem Aldehyd oder Keton, bevorzugt Keton, unter Verwendung eines Hydrierungskatalysators und bei einer Temperatur von 120 bis 150 °C erfolgt und Wasserstoff mit einem Druck von 35 bis 45 bar aufgedrückt wird und die Reaktion in einem Autoklaven oder in einem anderen Druckreaktor stattfindet.

12. Verfahren zur Herstellung der Verbindung gemäß Formel I), welches die folgenden Verfahrensschritte umfasst:
a2) Bereitstellung einer Verbindung gemäß Formel A2): 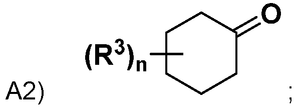 und
b2) Bereitstellung einer Verbindung gemäße Formel B2): 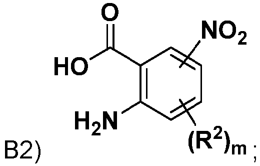 und
c2) Umsetzung der Verbindung gemäße Formel A2) mit der Verbindung gemäß Formel B2) in Gegenwart eines Halogenidüberträgers, wie PCl₃, POCl₃, PBr₃ oder SOCl₂, zu der Verbindung gemäß Formel C2): 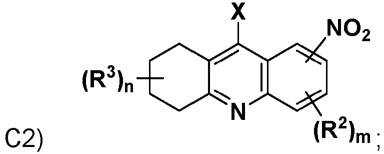 und
d2) Umsetzung der Verbindung gemäß Formel C2) in Gegenwart einer Säure, wie insbesondere Essigsäure, zu der Verbindung gemäß Formel B1): 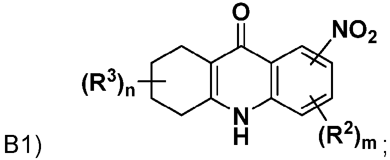 und
e2) Umsetzung der Verbindung gemäß Formel B1) mit Wasserstoff oder einem Hydrierungsreagenz, und einem Keton oder Aldehyd, zu der Verbindung gemäß Formel I): 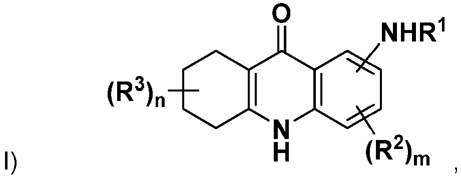 wobei R¹ ausgewählt ist aus der Gruppe bestehend aus Benzyl-Resten, 1-Phenylalkyl-Resten mit insgesamt 7 bis 18 Kohlenstoffatomen und linearen, verzweigten und cyclischen aliphatischen C₃- bis C₁₂-Resten; und wobei R³ ausgewählt ist aus der Gruppe bestehend aus linearen, verzweigten und cyclischen aliphatischen C₁- bis C₁₂-Resten, sowie Aryl-Resten, Ether-Resten und Thioether-Resten, und wobei n den Wert 0 oder 1 oder 2 oder 3 oder 4 annimmt, wobei die Reste R³ im Fall von n gleich 2 oder 3 oder 4 unabhängig voneinander gleich oder verschieden sind, und wobei R² ausgewählt ist aus der Gruppe bestehend aus linearen, verzweigten und cyclischen aliphatischen C₁- bis C₁₂-Resten, sowie Aryl-Resten, Ether-Resten und Thioether-Resten; und wobei m den Wert 0 oder 1 oder 2 oder 3 annimmt, wobei die Reste R² im Fall von m gleich 2 oder 3 unabhängig voneinander gleich oder verschieden sind, und wobei X ein Halogen, insbesondere Fluor (F), Chlor (Cl) oder Brom (Br), ist.

13. Verfahren nach Anspruch 12, **dadurch gekennzeichnet, dass** die Umsetzung in Schritt e2) mit Wasserstoff und dem Aldehyd oder Keton, bevorzugt Keton, unter Verwendung eines Hydrierungskatalysators und bei einer Temperatur von 50 bis 70 °C erfolgt und Wasserstoff mit einem Druck von 15 bis 25 bar aufgedrückt wird und die Reaktion in einem Autoklaven oder in einem anderen Druckreaktor stattfindet.

14. Kautschukmischung enthaltend die Verbindung nach einem der Ansprüche 1 bis 7, wobei die Kautschukmischung bevorzugt wenigstens einen Dienkautschuk enthält, der besonders bevorzugt ausgewählt ist aus der Gruppe bestehend aus natürlichem Polyisopren (NR), synthetischem Polyisopren (IR), Butadienkautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

15. Fahrzeugreifen, der die Kautschukmischung nach Anspruch 14 in wenigstens einem Bauteil, bevorzugt in wenigstens einem äußeren Bauteil, aufweist, wobei das äußere Bauteil bevorzugt ein Laufstreifen, eine Seitenwand und/oder ein Hornprofil ist.

## Claims

1. Compound of formula I): 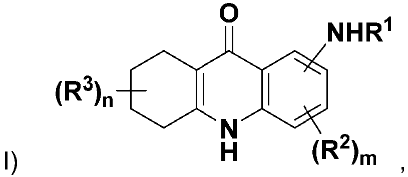
wherein R¹ is selected from the group consisting of benzyl radicals, 1-phenylalkyl radicals having altogether 7 to 18 carbon atoms and linear, branched and cyclic aliphatic C₃- to C₁₂-radicals; and
wherein R³ is selected from the group consisting of linear, branched and cyclic aliphatic C₁- to C₁₂-radicals, and aryl radicals, ether radicals and thioether radicals, and wherein n assumes the value 0 or 1 or 2 or 3 or 4, wherein when n is 2 or 3 or 4 the radicals R³ are independently of one another identical or different, and
wherein R² is selected from the group consisting of linear, branched and cyclic aliphatic C₁- to C₁₂-radicals, and aryl radicals, ether radicals and thioether radicals; and
wherein m assumes the value 0 or 1 or 2 or 3, wherein when m is 2 or 3 the radicals R² are independently of one another identical or different.

2. Compound according to Claim 1, **characterized in that** n is 0 (zero).

3. Compound according to Claim 1 or 2, **characterized in that** m is 0 (zero).

4. Compound according to any of the preceding claims, **characterized in that** R¹ is bonded to the nitrogen atom (N) via a tertiary carbon atom.

5. Compound according to any of the preceding claims, **characterized in that** R¹ is a branched alkyl radical having 3 to 12 carbon atoms, preferably 4 to 8 carbon atoms, or a 1-phenylalkyl radical having altogether 7 to 10 carbon atoms.

6. Compound according to any of the preceding claims, **characterized in that** R¹ is selected from the group consisting of 1,3-dimethylbutyl, 1-phenylethyl and cyclohexyl radicals, wherein R¹ is preferably a 1,3-dimethylbutyl radical.

7. Compound according to any of the preceding claims, **characterized in that** it has the structure of formula II):

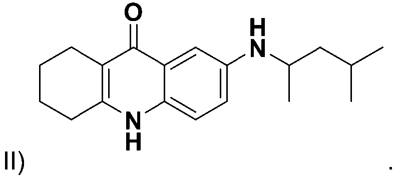

8. Use of the compound according to any of Claims 1 to 7 as an aging stabilizer, in particular in vehicle tyres or technical rubber articles, such as in particular an air spring, bellows, conveyor belt, belt, drive belt, hose, rubber band, profile, a seal, a membrane, tactile sensors for medical applications or robotics applications, or a shoe sole or parts thereof, and/or oils and/or lubricants.

9. Use of the compound according to any of Claims 1 to 7 as a dye in fibres and/or polymers and/or paper and/or in (decorating) paints and coatings.

10. Process for producing the compound of formula I) which comprises the following process steps:
a1) providing the compound of formula A1) 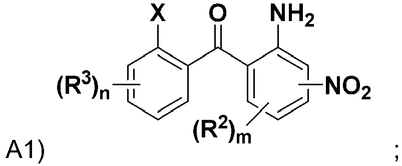
b1) reacting the compound of formula A1) with a base, in particular potassium carbonate (K₂CO₃), to obtain the compound of formula B1): 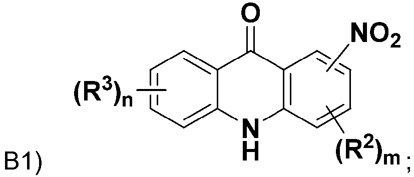
c1) reacting the compound of formula B1) with hydrogen or a hydrogenating reagent, and a ketone or aldehyde, to afford the compound of formula I): 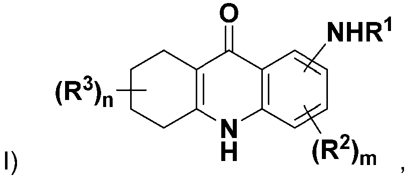
wherein R¹ is selected from the group consisting of benzyl radicals, 1-phenylalkyl radicals having altogether 7 to 18 carbon atoms and linear, branched and cyclic aliphatic C₃- to C₁₂-radicals; and
wherein R³ is selected from the group consisting of linear, branched and cyclic aliphatic C₁- to C₁₂-radicals, and aryl radicals, ether radicals and thioether radicals, and wherein n assumes the value 0 or 1 or 2 or 3 or 4, wherein when n is 2 or 3 or 4 the radicals R³ are independently of one another identical or different, and
wherein R² is selected from the group consisting of linear, branched and cyclic aliphatic C₁- to C₁₂-radicals, and aryl radicals, ether radicals and thioether radicals; and
wherein m assumes the value 0 or 1 or 2 or 3, wherein when m is 2 or 3 the radicals R² are independently of one another identical or different, and wherein X is a halogen, in particular fluorine (F), chlorine (CI) or bromine (Br).

11. Process according to Claim 10, **characterized in that** the reaction in step c1) with hydrogen and the aldehyde or ketone, preferably ketone, is carried out using a hydrogenation catalyst and at a temperature of 120°C to 150°C and the reaction mixture is subjected to hydrogen at a pressure of 35 to 45 bar and the reaction is carried out in an autoclave or in another pressure reactor.

12. Process for producing the compound of formula I) which comprises the following process steps:
a2) providing a compound of formula A2): 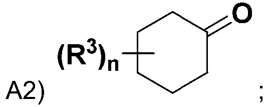 and
b2) providing a compound of formula B2): 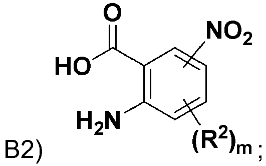 and
c2) reacting the compound of formula A2) with the compound of formula B2) in the presence of a halogenating agent such as PCl₃, POCl₃, PBr₃ or SOCl₂ to afford the compound of formula C2): 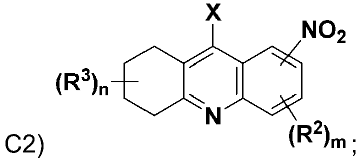 and
d2) reacting the compound of formula C2) in the presence of an acid, such as in particular acetic acid, to afford the compound of formula B1): 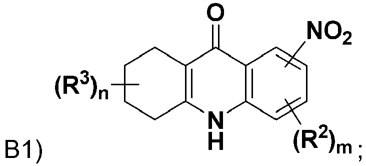 and
e2) reacting the compound of formula B1) with hydrogen or a hydrogenating reagent, and a ketone or aldehyde, to afford the compound of formula I): 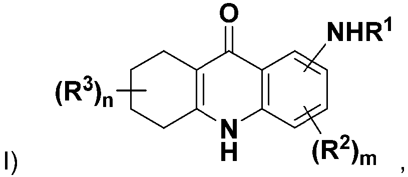
wherein R¹ is selected from the group consisting of benzyl radicals, 1-phenylalkyl radicals having altogether 7 to 18 carbon atoms and linear, branched and cyclic aliphatic C₃- to C₁₂-radicals; and
wherein R³ is selected from the group consisting of linear, branched and cyclic aliphatic C₁- to C₁₂-radicals, and aryl radicals, ether radicals and thioether radicals,
and wherein n assumes the value 0 or 1 or 2 or 3 or 4, wherein when n is 2 or 3 or 4 the radicals R³ are independently of one another identical or different, and
wherein R² is selected from the group consisting of linear, branched and cyclic aliphatic C₁- to C₁₂-radicals, and aryl radicals, ether radicals and thioether radicals; and
wherein m assumes the value 0 or 1 or 2 or 3, wherein when m is 2 or 3 the radicals R² are independently of one another identical or different, and wherein X is a halogen, in particular fluorine (F), chlorine (CI) or bromine (Br).

13. Process according to Claim 12, **characterized in that** the reaction in step e2) with hydrogen and the aldehyde or ketone, preferably ketone, is carried out using a hydrogenation catalyst and at a temperature of 50°C to 70°C and the reaction mixture is subjected to hydrogen at a pressure of 15 to 25 bar and the reaction is carried out in an autoclave or in another pressure reactor.

14. Rubber mixture containing the compound according to any of Claims 1 to 7, wherein the rubber mixture preferably contains at least one diene rubber which is particularly preferably selected from the group consisting of natural polyisoprene (NR), synthetic polyisoprene (IR), butadiene rubber (BR), solution-polymerized styrene-butadiene rubber (SSBR), emulsion-polymerized styrene-butadiene rubber (ESBR), butyl rubber (IIR) and halobutyl rubber.

15. Vehicle tyre comprising the rubber mixture according to Claim 14 in at least one component, preferably in at least one outer component, wherein the outer component is preferably a tread, a sidewall and/or a flange profile.

## Revendications

1. Composé selon la formule I) : 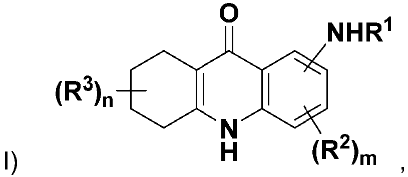
dans laquelle R¹ est choisi dans le groupe constitué par les radicaux benzyle, les radicaux 1-phénylalkyle comprenant au total 7 à 18 atomes de carbone et les radicaux aliphatiques linéaires, ramifiés et cycliques en C₃-C₁₂ ; et
dans laquelle R³ est choisi dans le groupe constitué par les radicaux aliphatiques linéaires, ramifiés et cycliques en C₁-C₁₂, ainsi que les radicaux aryle, les radicaux éther et les radicaux thioéther et dans laquelle n prend la valeur 0 ou 1 ou 2 ou 3 ou 4, les radicaux R³ étant identiques ou différents, indépendamment les uns des autres, dans le cas où n vaut 2 ou 3 ou 4 et
dans laquelle R² est choisi dans le groupe constitué par les radicaux aliphatiques linéaires, ramifiés et cycliques en C₁-C₁₂ ainsi que les radicaux aryle, les radicaux éther et les radicaux thioéther ; et
dans laquelle m prend la valeur 0 ou 1 ou 2 ou 3, les radicaux R² étant identiques ou différents, indépendamment les uns des autres, dans le cas où m vaut 2 ou 3.

2. Composé selon la revendication 1, **caractérisé en ce que** n vaut 0 (zéro).

3. Composé selon la revendication 1 ou 2, **caractérisé en ce que** m vaut 0 (zéro).

4. Composé selon l'une des revendications précédentes, **caractérisé en ce que** R¹ est lié à l'atome d'azote (N) par l'intermédiaire d'un atome de carbone tertiaire.

5. Composé selon l'une des revendications précédentes, **caractérisé en ce que** R¹ représente un radical alkyle ramifié comprenant de 3 à 12 atomes de carbone, de préférence 4 à 8 atomes de carbone, ou un radical 1-phénylalkyle comprenant au total 7 à 10 atomes de carbone.

6. Composé selon l'une des revendications précédentes, **caractérisé en ce que** R¹ est choisi dans le groupe constitué par les radicaux 1,3-diméthylbutyle, 1-phényléthyle et cyclohexyle, R¹ étant de préférence un radical 1,3-diméthylbutyle.

7. Composé selon l'une des revendications précédentes, **caractérisé en ce qu'**il présente la structure selon la formule II) :

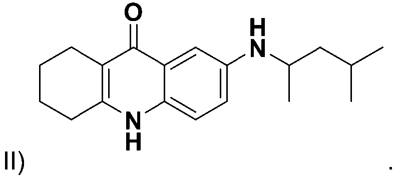

8. Utilisation du composé selon l'une des revendications 1 à 7 en tant qu'agent de protection contre le vieillissement, en particulier dans des pneumatiques pour véhicule ou d'autres articles techniques en caoutchouc, tels qu'en particulier un ressort pneumatique, un soufflet, une bande transporteuse, une sangle, une courroie, un tuyau, une bande en caoutchouc, un profilé, un joint d'étanchéité, une membrane, des capteurs tactiles pour applications médicales ou applications robotiques, ou une semelle de chaussure ou de parties de celle-ci, et/ou des huiles et/ou des lubrifiants.

9. Utilisation du composé selon l'une des revendications 1 à 7 en tant que colorant dans des fibres et/ou des polymères et/ou du papier et/ou des peintures et vernis (d'enduction).

10. Procédé pour la préparation du composé selon la formule I), qui comprend les étapes de procédé suivantes :
a1) mise à disposition de la substance selon la formule A1) 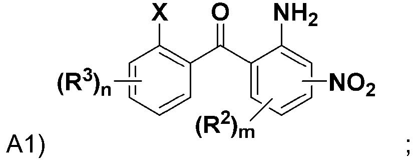
b1) transformation du composé selon la formule A1) avec une base, en particulier du carbonate de potassium (K₂CO₃), le composé selon la formule B1) étant obtenu : 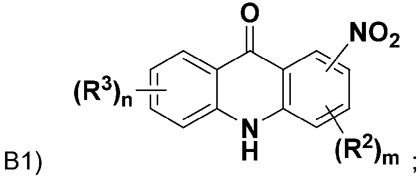
c1) transformation du composé selon la formule B1) avec de l'hydrogène ou un réactif d'hydrogénation et une cétone ou un aldéhyde en composé selon la formule I) : 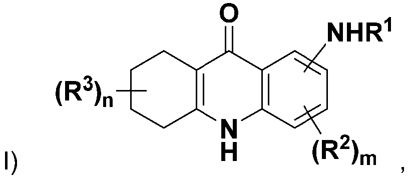
dans laquelle R¹ est choisi dans le groupe constitué par les radicaux benzyle, les radicaux 1-phénylalkyle comprenant au total 7 à 18 atomes de carbone et les radicaux aliphatiques linéaires, ramifiés et cycliques en C₃-C₁₂ ; et
dans laquelle R³ est choisi dans le groupe constitué par les radicaux aliphatiques linéaires, ramifiés et cycliques en C₁-C₁₂, ainsi que les radicaux aryle, les radicaux éther et les radicaux thioéther et dans laquelle n prend la valeur 0 ou 1 ou 2 ou 3 ou 4, les radicaux R³ étant identiques ou différents, indépendamment les uns des autres, dans le cas où n vaut 2 ou 3 ou 4 et
dans laquelle R² est choisi dans le groupe constitué par les radicaux aliphatiques linéaires, ramifiés et cycliques en C₁-C₁₂ ainsi que les radicaux aryle, les radicaux éther et les radicaux thioéther ; et
dans laquelle m prend la valeur 0 ou 1 ou 2 ou 3, les radicaux R² étant identiques ou différents, indépendamment les uns des autres, dans le cas où m vaut 2 ou 3, et dans laquelle X représente halogène, en particulier fluor (F), chlore (CI) ou brome (Br).

11. Procédé selon la revendication 10, **caractérisé en ce que** la transformation dans l'étape c1) est effectuée avec de l'hydrogène et l'aldéhyde ou la cétone, de préférence la cétone, avec utilisation d'un catalyseur d'hydrogénation et à une température de 120 à 150°C et l'hydrogène est comprimé à une pression de 35 à 45 bars et la réaction a lieu dans un autoclave ou dans un autre réacteur sous pression.

12. Procédé pour la préparation du composé selon la formule I), qui comprend les étapes de procédé suivantes :
a2) mise à disposition d'un composé selon la formule A2) : 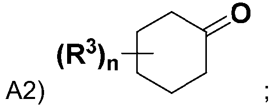 et
b2) mise à disposition d'un composé selon la formule B2) : 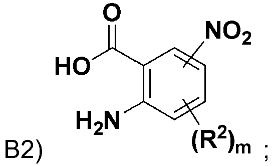 et
c2) transformation du composé selon la formule A2) avec le composé selon la formule B2) en présence d'un agent de transfert d'halogénure, tel que PCl₃, POCl₃, PBr₃ ou SOCl₂, en composé selon la formule C2) : 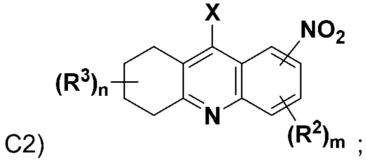 et
d2) transformation du composé selon la formule C2) en présence d'un acide, tel qu'en particulier l'acide acétique, en composé selon la formule B1) : 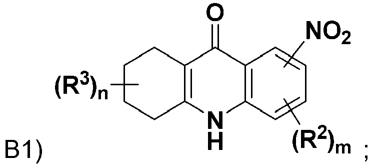 et
e2) transformation du composé selon la formule B1) avec de l'hydrogène ou un réactif d'hydrogénation et une cétone ou un aldéhyde en composé selon la formule I) : 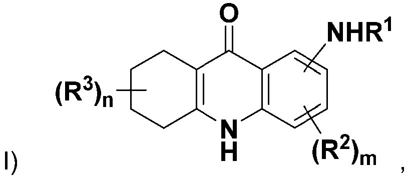
dans laquelle R¹ est choisi dans le groupe constitué par les radicaux benzyle, les radicaux 1-phénylalkyle comprenant au total 7 à 18 atomes de carbone et les radicaux aliphatiques linéaires, ramifiés et cycliques en C₃-C₁₂ ; et
dans laquelle R³ est choisi dans le groupe constitué par les radicaux aliphatiques linéaires, ramifiés et cycliques en C₁-C₁₂, ainsi que les radicaux aryle, les radicaux éther et les radicaux thioéther et dans laquelle n prend la valeur 0 ou 1 ou 2 ou 3 ou 4, les radicaux R³ étant identiques ou différents, indépendamment les uns des autres, dans le cas où n vaut 2 ou 3 ou 4 et
dans laquelle R² est choisi dans le groupe constitué par les radicaux aliphatiques linéaires, ramifiés et cycliques en C₁-C₁₂ ainsi que les radicaux aryle, les radicaux éther et les radicaux thioéther ; et
dans laquelle m prend la valeur 0 ou 1 ou 2 ou 3, les radicaux R² étant identiques ou différents, indépendamment les uns des autres, dans le cas où m vaut 2 ou 3, et dans laquelle X représente halogène, en particulier fluor (F), chlore (CI) ou brome (Br).

13. Procédé selon la revendication 12, **caractérisé en ce que** la transformation dans l'étape e2) est effectuée avec de l'hydrogène et l'aldéhyde ou la cétone, de préférence la cétone, avec utilisation d'un catalyseur d'hydrogénation et à une température de 50 à 70°C et l'hydrogène est comprimé à une pression de 15 à 25 bars et la réaction a lieu dans un autoclave ou dans un autre réacteur sous pression.

14. Composition de caoutchouc contenant le composé selon l'une des revendications 1 à 7, la composition de caoutchouc contenant de préférence au moins un caoutchouc diénique, qui est de manière particulièrement préférée choisi dans le groupe constitué par le polyisoprène naturel (NR), le polyisoprène synthétique (IR), le caoutchouc butadiène (BR), le caoutchouc styrène-butadiène polymérisé en solution (SSBR), le caoutchouc styrène-butadiène polymérisé en émulsion (ESBR), le caoutchouc butyle (IIR) et le caoutchouc halogénobutyle.

15. Pneumatique pour véhicule, qui présente le mélange de caoutchouc selon la revendication 14 dans au moins un composant, de préférence dans au moins un composant externe, le composant externe étant de préférence une bande de roulement, un flanc latéral et/ou un profilé de talon.
